(19) Europäisches Patentamt
European Patent Office
Office européen des brevets

(11) **EP 3 081 256 B1**

(12) **EUROPEAN PATENT SPECIFICATION**

(45) Date of publication and mention
of the grant of the patent:
**27.10.2021 Bulletin 2021/43**

(51) Int Cl.:
*A61N 1/08* *(2006.01)*          *A61N 1/36* *(2006.01)*
*A61N 1/04* *(2006.01)*

(21) Application number: **14869518.2**

(22) Date of filing: **28.11.2014**

(86) International application number:
**PCT/ES2014/070878**

(87) International publication number:
**WO 2015/086873 (18.06.2015 Gazette 2015/24)**

(54) **ELECTROTHERAPY DEVICE**

ELEKTROTHERAPIEVORRICHTUNG

DISPOSITIF D'ÉLECTROTHÉRAPIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.12.2013 ES 201331817**

(43) Date of publication of application:
**19.10.2016 Bulletin 2016/42**

(73) Proprietor: **Indiba, S.A.**
**08192 Sant Quirze del Valles (Barcelona) (ES)**

(72) Inventor: **RAMI MURILLO, Xavier**
**08032 Barcelona (ES)**

(74) Representative: **Durán-Corretjer, S.L.P.**
**Còrsega, 329**
**(Paseo de Gracia/Diagonal)**
**08037 Barcelona (ES)**

(56) References cited:
**WO-A1-2013/059158          ES-B1- 2 304 272**
**ES-U- 1 030 072          FR-A1- 2 973 709**
**US-A- 5 891 185          US-A1- 2002 082 653**
**US-A1- 2003 139 781          US-A1- 2005 065 554**
**US-A1- 2010 152 817          US-B1- 6 516 227**

• **DATABASE WPI Week 199844, Derwent
Publications Ltd., London, GB; Class P34, AN
1998-518902, XP055349260 THOMSON & RU 2 106
885 C1 (CHERKASOV YUM) 20 March 1998**

**Description**

The present invention relates to an electrotherapy device applicable to living tissue, said electrotherapy being moderate diathermy produced by radiofrequency (RF) electric currents applied by means of contact electrodes. In particular, said electrotherapy is performed at neurologically active vascularised points on the patient.

The neurologically active vascularised points are related to a stretch of the connective tissue of the hypodermis which conveys vessel and nerve elements for the skin. 42 % of the neurologically active vascularised points are located on known nerves or very close thereto. Others are located on major blood vessels or very close thereto (18 % on arteries and 40 % on veins) . Said blood vessels are surrounded by small nerve bundles forming the nervi vasorum. The nature of these nerve bundles, which are found beneath the neurologically active vascularised point, is varied: cutaneous bundles (which are purely sensory or sensory and sympathetic), vascular bundles (a mixture of sympathetic and sensory) or muscular bundles (a mixture of sensory and motor).

The production of afferent influence on the peripheral nerves is vital for the control of pain with electric currents; a suitable place for the application of a current is the point where the cutaneous nerve penetrates the fascia. A similar appraisal can also be made for the motor points, which have the common anatomical characteristic of being the points through which the nerve penetrates the muscle.

Many diathermy devices are known in the prior art. Diathermy is a technique which uses high frequency currents (less than 100 kHz) applied by means of an electrode to produce local heating of the cellular tissues of particular parts of the body affected by ailments, for example. Said diathermy devices result in heating of the tissues but do not produce electro-stimulation.

In general, diathermy equipment increases the temperature of the internal tissues by causing currents that can be as high as 3 A to pass through.

Some diathermy devices use currents controlled by pulse-width modulation (PWM), and in this case higher currents may be used.

The increase in temperature of the living tissue by diathermy is achieved by transmitting energy thereto by two methods: induced currents (electrodes not in contact with the tissue) or conducted currents (electrodes in contact with the tissue) . Unlike transcutaneous electrical nerve stimulation (or TENS) devices, electrotherapy devices that function with RF currents of more than 100 kHz, such as diathermy equipment, do not produce electro-stimulation of the nerves. In general, the frequency of the signal applied in the contactless coupling method must be far higher than the frequency of the signal applied in the contact coupling method, said frequencies in fact being over 100 kHz. Further details of the effects of electric currents on human beings and livestock have already been studied and are regulated by the IEC 60479 standards.

In conducted current diathermy two electrodes are applied in contact with the living tissue in order to produce a circulation of electric current that passes through the tissue found in its path. Due to the electrical impedance of said tissue, the electric current that circulates through the tissue causes a rise in the temperature thereof through the Joule effect.

Unlike in the conventional use of diathermy equipment which, due to the very strong current that is applied, generally requires constant movement of the active electrode on the tissue being treated, treatment of neurologically active vascularised points is carried out using electric currents of approximately a few milliamperes for a few minutes and with the active electrode static and in contact with the neurologically active vascularised point.

Present examples of diathermy equipment by conduction have an active electrode and a return electrode, as disclosed for example in patents ES 287 964 and EP 0 893 140.

These devices are intended for the therapeutic treatment of defined affected zones. One of the differences between these devices and that of the present invention lies in the functionality that the present invention discloses for the simultaneous treatment of multiple zones with the possibility of using different parameters for voltage, current and/or frequency, for example, at each zone.

A similar solution is disclosed in document US 2008 0015572 for the treatment of acupuncture points in which the active electrodes are needles. With this configuration, the therapist can in no circumstances select the electric current for the treatment of each neurologically active vascularised point, as each needle is connected to the same generator.

Documents ES1030072 and ES2304272 disclose particular embodiments of diathermy devices which comprise pairs of electrodes connected to independent generators. However, the treatment of neurologically active vascularised points with this type of device would require a pair of electrodes for each of the neurologically active vascularised points. This would not only cause the difficulty of having to position the active electrode at each neurologically active vascularised point, but the spot where each return electrode should be positioned would also have to be determined precisely.

As it is unique, each neurologically active vascularised point must be treated with a current that has an amplitude that is independent of the others. Thus one of the problems to be solved by the present invention is how to treat different neurologically active vascularised points with a single device.

Document US2002/0082653 discloses a pacemaker. Said pacemakers are classified in the industry sector as "electromedical apparatus" to differentiate said pacemakers from "electrotherapy apparatus". Pacemakers are small electronic devices that discontinuously and rhythmically (using bipolar electrodes) excite a heart that is unable to contract regularly for itself, the electrodes being situated in the heart. The bipolar electrodes function simultaneously as anode and cathode and are incorporated in a single physical unit at a single location.

Document US2003/139781 A1 discloses a method for determining the relative position and orientation of electrodes on a neurostimulation lead or leads used with a neurostimulation system.

Document US2010/152817 A1 discloses a device for applying pulses to a patient. Document WO2013/059158 A1 discloses a muscular stimulation device.

The present invention relates to a device that performs moderate diathermy therapy by means of at least two active electrodes and one return electrode, with the possibility of monitoring and/or varying the amplitude, frequency and phase of each of the generators connected to each of the active electrodes.

The electrodes of the present invention are preferably electrodes for application to the skin, divided into active electrodes and return electrodes.

The return electrodes may preferably be a single ring-shaped plate, also known as neutral electrodes in which the return plate allows ions to be returned to the active electrode.

The active electrodes are preferably disk-shaped surfaces for application to the skin surface, although needle-shaped active electrodes which penetrate the skin tissue are also possible.

The active electrodes can be differentiated into two types according to the use thereof: the capacitive electrode, which is suitable for superficial and vascularised tissue and the resistive electrode, which is suitable for thick, fatty and fibrotic tissue. Preferably the active electrodes are conducting electrodes with no insulating layer.

The present invention relates to an electrotherapy device which comprises:

- a plurality of active electrodes;

- a return electrode; and

- a plurality of voltage generators each connected to an active electrode;

in which each device comprises a voltage generator controller which has means for monitoring and/or varying the voltage supplied to each of the active electrodes independently according to claim 1.

In a particular embodiment of the present invention the controller comprises means for monitoring and/or varying the phase and/or frequency of the voltage supplied to each of the active electrodes, as well as the voltage.

Preferably, the maximum values said electrotherapy device reaches for each of the current outputs of the generator are: current of 300 mA RMS, voltage of 70 V RMS and/or electric power of 50 W.

Furthermore, the output signal of each generator is preferably sinusoidal with a harmonic distortion of less than 50 % and with a frequency of between 100 kHz and 2 MHz.

Moreover, to be able to monitor and/or vary each of the generator outputs individually, the controller may be a digital controller which comprises a microcontroller or a microprocessor. In other embodiments, the controller may be a programmable logic circuit from among those known in the prior art, such as a field programmable gate array (FPGA) or a complex programmable logic device (CPLD).

In particular embodiments of the present invention the controller could be an analogue circuit.

To give the device greater flexibility, at least one of the electrodes may comprise a commutator which switches the active electrode from a first position connected to the output of the generator to a second position connected to the return electrode. Thus, in the first position, the electrode would be an active electrode and in the second position the electrode would still be configured as a return electrode.

More preferably, at least one of the electrodes comprises a temperature sensor.

Preferably the device comprises a single return electrode.

In particular, the active electrodes comprise connection means to the patient, said means possibly being adhesive means or suction means, for example, among others.

For a better understanding the accompanying drawings show an embodiment of the device of the present invention as an explanatory but not limiting example.

Fig. 1 is an electrical diagram of an embodiment of a device according to the present invention.

Fig. 2 is an electrical diagram of a second embodiment of a device according to the present invention with two active electrodes.

Fig. 3 is an electrical diagram of a third embodiment of a device according to the present invention with three active electrodes.

Fig. 4 is a flow diagram of the method of monitoring and/or varying the controller of the signal generator.

Fig. 5 shows, by way of example, some of the neurologically active vascularised points of the human body.

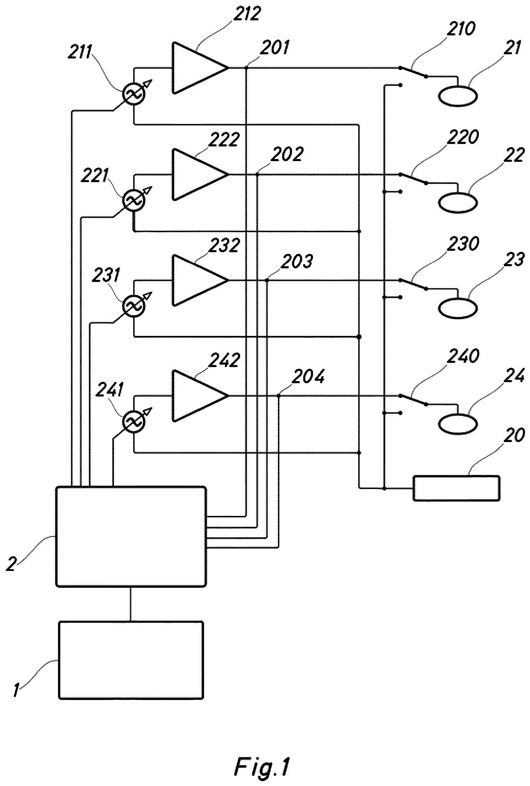

Fig. 1 is a diagram of a device according to the present invention. Said device has four active electrodes -21-, -22-, -23-, -24- and a single return electrode -20-.

To supply current to the active electrodes -21-, -22-, -23-, -24-, the device comprises multiple independent voltage generators -211-, -221-, -231-, -241- which can be regulated individually and controlled by the controller -2-. Furthermore, it is possible to arrange amplifiers -212-, -222-, -232-, -242- at the output of said generators in some embodiments of the present invention.

In addition, said controller -2- comprises control means that allow the frequency, phase and amplitude of the output signal of each of the generators to be monitored and/or varied. Said monitoring and/or variation of the output signals of the generators can be carried out using analogue control circuits (by means of operational amplifiers or similar) or digital control circuits (such as microprocessors, microcontrollers, FPGAs, CPLDs, among others).

Furthermore, to select the parameters of the treatment to be carried out, data acquisition means -1- are provided. Said data acquisition means may be analogue means (for example, potentiometers) or digital means (such as switches, touch screens, etc.).

For optimal functioning of the controller -2-, it is vital to have a true measurement of the current that is circulating through each active electrode. The present invention envisages an arrangement of current measurement devices at points -201-, -202-, -203-, -204- at the output of the amplifiers or generators depending on the configuration of the device.

Because the neurologically active vascularised points are fixed points, the active electrodes -21-, -22-, -23-, -24- must be in contact with the tissue and remain fixed during therapy. Consequently, said electrodes may be of the adhesive patch, suction or equivalent type, for example, and the active surface thereof is preferably metallic.

Furthermore, said arrangement of fixed electrodes suggests the provision of means for allowing the electric power to be limited by limiting the current and/or maximum voltage applied at each point so as not to damage the tissues by an excessive rise in temperature.

In a preferred embodiment, the area of the active electrode should be similar to the area of the neurologically active vascularised points so that the maximum electric current selected by the therapist passes through the neurologically active vascularised point. It has been determined that the ideal area of the active electrode for treatment of the neurologically active vascularised points is, at most, 2 cm$^2$.

To reduce the impedance between the active electrodes and the return electrode, at least one of the active electrodes -21-, -22-, -23-, -24- may be commutated in order to be converted into a return electrode. This is achieved by the arrangement of commutators -210-, -220-, -230-, -240- for selecting whether the active electrode is connected to the voltage generator (and, thus, to act as an active electrode) or to the return electrode (to act as a return electrode) .

In addition, each electrode may have a temperature sensor (not shown) to monitor the temperature of the electrode or the skin.

In a particular embodiment, as can be seen in Fig. 1, Fig. 2 and Fig. 3, the device comprises a single return electrode -20-.

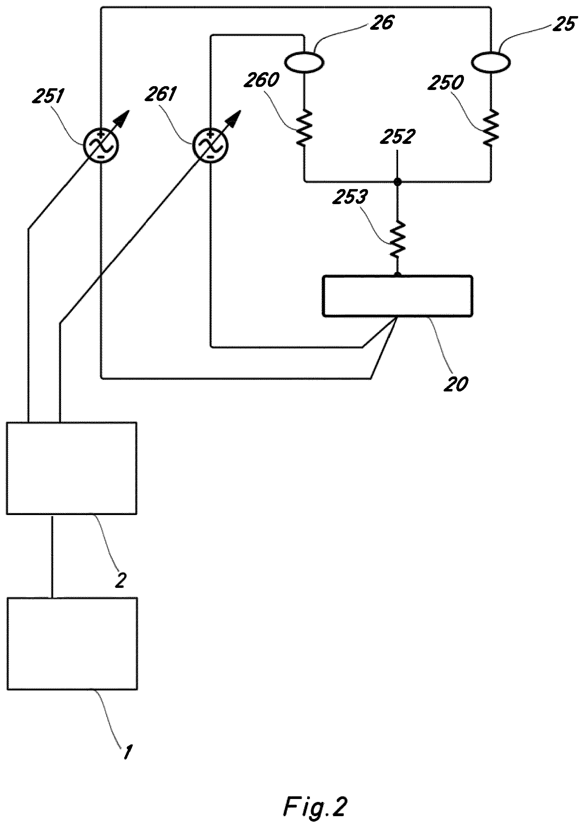

Fig. 2 shows an electrical impedances model simulating an embodiment of the present invention which comprises two active electrodes. In this figure, the use of a first voltage generator -251- associated with a first active electrode -25- and a second voltage generator -261- associated with a second active electrode -26- can be seen. Furthermore there is a return electrode -20- which closes the circuit.

One of the problems that must be solved in the present invention is that, as can be seen in the figure, human tissue offers a series resistance -250-, -260- at the output of each electrode -25-, -26- which would allow easy calculation of the voltage that should be applied to each electrode to obtain a particular current through the tissue; however, the existence of a common resistance -253- (which is inherent to the tissue) means that the output current of the electrodes -25-, -26- is located at a common point -252- causing the output currents to interfere with each other.

A possible solution would be to arrange a device for measuring the current that circulates through each of the electrodes -25-, -26- and modify the voltage of at least one of the generators iteratively until the required current is obtained at each of the electrodes -25-, -26-. This voltage modification can be carried out by automatic means or manually at each of the generators -251-, -261-.

For example, in Fig. 2 the required current ($I_{-250-}$) through the electrode -25- is 20 mA and the current ($I_{-260-}$) through the electrode -26- should be 30 mA. The impedance of the tissues at 448 kHz is basically resistive, and the reactive portion thereof can therefore be disregarded. An example of the values of the equivalent impedances could be:

Impedance at the first series resistance -250-:

$Z_{-250-}$ = 300 ohm
Impedance at the second series resistance -260-:
$Z_{-260-}$ = 600 ohm
Impedance at the common resistance -253-:
$Z_{-253-}$ = 500 ohm

The voltages necessary of the RF generators -251-, and -261-for $I_{-250-}$ to be 20 mA and $I_{-260-}$ to be 30 mA are:

Voltage at the first generator -251-: $V_{-251-}$ = 31 V
Voltage at the second generator -261-: $V_{-261-}$ = 43 V
With a voltage in common mode (at point -253-) of:
$V_{-253-}$ = 25 V

In this example, as the common impedance -253- is relatively high, it results in the input voltages also being high.

If only the electrode -25- is applied, the voltage $V_{-251-}$ necessary for $I_{-250-}$ = 20 mA would be 16 V, and if only the electrode -26- is applied for $I_{-260-}$ = 30 mA to hold, the voltage $V_{-261-}$ would be 33 V; less than 31 V and 43 V when both electrodes are applied together.

However, it is not always possible to reduce the value of the common impedance because said impedance depends on the composition of the tissues and the position of the electrodes.

The effect of the common impedance means that the current of each electrode depends on the currents of the other electrodes. This problem can be better understood by referring to the previous example in which a simplified example with two RF generators connected to two active electrodes is shown. In this case, it holds that:

$$I_{-250-} = \frac{V_{-251-} - V_{-253-}}{Z_{-250-}}$$

$$I_{-260-} = \frac{V_{-261-} - V_{-253-}}{Z_{-260-}}$$

$$V_{-253-} = Z_{-253-} \cdot \left( I_{-290-} + I_{-260-} \right)$$

The currents $I_{-250-}$ and $I_{-260-}$ depend on the common voltage $V_{-253-}$, which in turn depends on the value of the currents $I_{-250-}$ and $I_{-260-}$.

If for example $I_{-250-}$ is fixed, on increasing the value of $I_{-260-}$, the common voltage $V_{-253-}$ will increase, and this will cause the voltage between the ends of the impedance $Z_{-250-}$ to diminish, reducing the value of the current $I_{-250-}$. If the voltage $V_{-251-}$ is increased to compensate for this fall, the common voltage $V_{-253-}$ will increase in the same way, reducing the value of $I_{-260-}$.

Another example that further exacerbates this problem is when the return electrode is at one of the patient's extremities, for example on the hand or leg; in this case the common impedance $Z_{-253-}$ may be at the maximum. The result is that some neurologically active vascularised points would be treated excessively and at others there would be a deficit.

The only way to avoid this dependency is for the common impedance $Z_{-253-}$ where the currents $I_{-250-}$ and $I_{-260-}$ are added together, to tend to zero.

An alternative proposed by the present invention to reduce the common voltage is by modifying the phase of the currents of the electrodes, so that they tend to cancel each other out causing a reduction in the voltage at the common impedance $Z_{-253-}$. In this case we would also have:

$$V_{-251-}(t) = V_1 \cdot \sin \omega t + \varphi_1)$$

$$V_{-261-}(t) = V_2 \cdot \sin \omega t + \varphi_2)$$

$$V_{-253-}(t) = Z_{-253-} \cdot [I_{-250-}(t) + I_{-260-}(t)]$$

If for example the voltage $V_{-261-}$ is out of phase by 180° with respect to the phase $V_{-251-}$ with $\varphi_1$, = 0° and $\varphi_2$ = 180°, for the same parameters of $I_{-250-}$, $I_{-260-}$, $Z_{-250-}$, $Z_{-260-}$ and $Z_{-253-}$, selected in the previous example, the currents $I_{-250-}$, $I_{-260-}$ will remain, reducing the value of the common voltage $V_{-253-}$.

The voltages required are:

$$V_{-251-} = 1 \text{ V},$$

and

$$V_{-261-} = 23 \text{ V} \angle 180°$$

with a voltage in common mode of $V_{-253-}$ = 5 V∠180°.

In this example, a phase variation of 180° has been shown, but it could be any other phase between 0° and ±180°. By varying the phases of the signals, the voltage in common mode is reduced, and therefore lower voltages can be applied to achieve the same therapeutic current at the neurologically active vascularised points. Consequently, the electrical power dissipated by the tissues that are not being treated is also reduced, as the aim is not deep treatment as in profound diathermy, but rather treatment close to the neurologically active vascularised points, which are in the hypodermis.

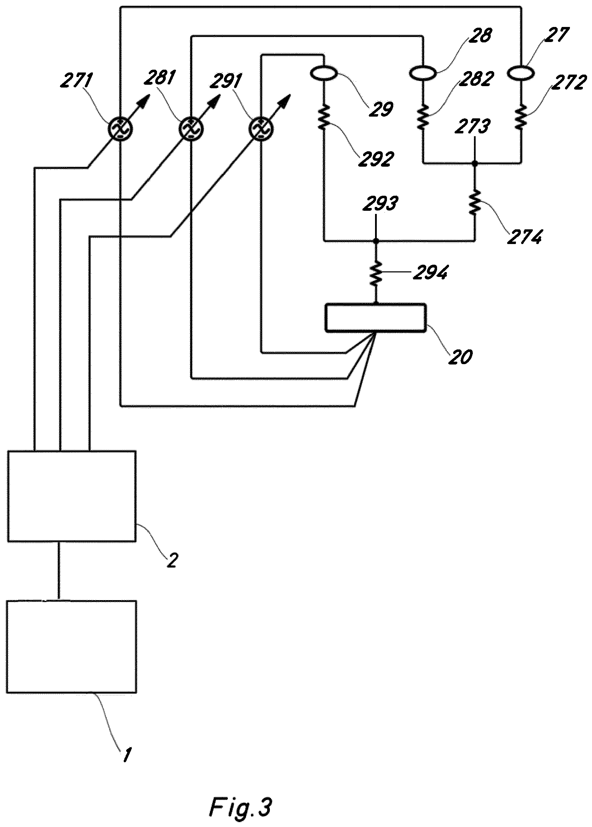

In an embodiment like that shown in Fig. 3, in which there are three active electrodes -27-, -28-, -29- each connected to an independent generator -271-, -281-, -291- there are more variables to control, since there is a series resistance -272-, -282-, -292- for each of the electrodes -27-, -28-, -29- and two common resistances -274-, -294- which define two common points -273-, -293- which obstruct said iterative process at each of the generators to obtain the required current through each neurologically active vascularised point.

This simplified electrical model can be scaled to embodiments in which there are more than three electrodes, bearing in mind that new common impedances appear between the electrodes. With the present invention, twelve neurologically active vascularised points for example can be treated simultaneously and it is therefore necessary to have a controller that adjusts the voltage, phase and frequency of each RF generator, so that the current selected by the therapist circulates through each active electrode.

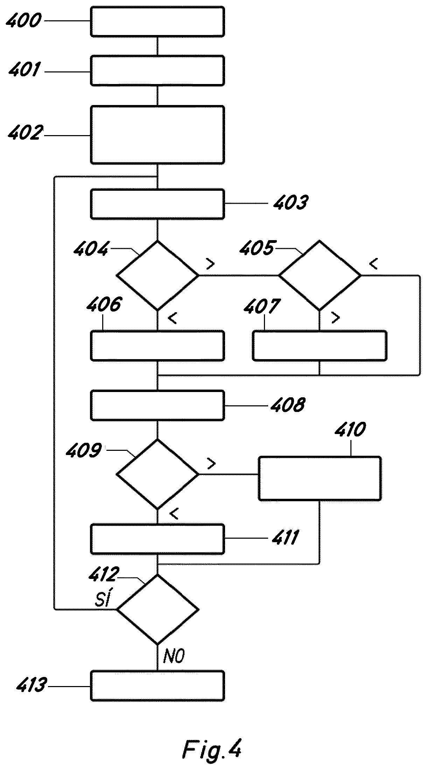

A flow diagram of a proposed controller is shown in Fig. 4. The treatment parameters -400- are selected in said controller, the impedances at the output of each electrode -401- are measured and, once the controller has the value of said parameters available, it actuates the generator -402- (or group of generators) in order to achieve at the output the required treatment current at each electrode. This takes place for all the outputs.

Next, a second measurement -403- is carried out of the current at each of the outputs. If the measured current for each of the electrodes (allowing some tolerance, preferably 10 %) is less than the required current -404- the voltage from the generator -406- must be increased, if it is greater, it must be asked if the output current is greater than the required current -405- (allowing some tolerance).

If the current is greater than the required current, the voltage of the generator -407- must be reduced. Once the modifications have been carried out at the generators (if necessary) there is a pause -408- to stabilise the voltage and current measurements.

Following this pause a measurement is taken of the output voltages -409-. If the output voltage is greater than the maximum permitted voltage (or is close thereto) the voltage of the generator must be reduced and the phase of the signal -410- modified. Thus, modification of the phase allows greater currents, including applying a lower voltage because alternating current signals are added together.

Once the required current is achieved at an electrode the process passes to the next channel -411-, corresponding to the next electrode.

After passing to the next channel, the controller asks if the treatment -412- is to continue and, if affirmative, carries out the second current measurement -409- and continues the process. If a signal to terminate treatment arrives, all the generators -413- are deactivated.

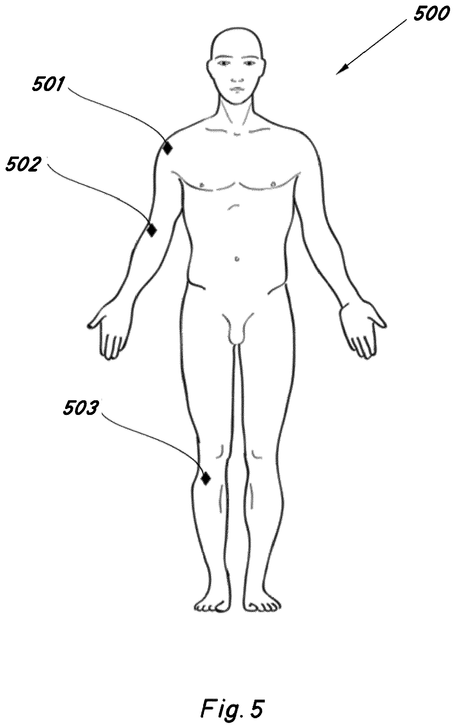

Fig. 5 shows an example of neurologically active vascularised points located on the human body -500-. The inventors of the present invention have located over a thousand neurologically active vascularised points in humans; however, to give an example, neurologically active vascularised points have been shown located at the shoulder -501-, at the front part of the elbow -502- and below the knees -503-.

Although the invention has been described with respect to preferred embodiments, said embodiments should not be considered as limiting the invention, which will be defined by the widest interpretation of the following claims.

**Claims**

1. Electrotherapy device which comprises:

   - a plurality of active electrodes;
   - a return electrode; and
   - a plurality of voltage generators each connected to an active electrode;

   said device comprising a voltage generator controller which has means for monitoring and/or varying the voltage supplied to each of the active electrodes independently;
   **characterised in that** the voltage in common mode at the common resistance is reduced by modifying the phase of the currents of the electrodes or by modifying the voltage of at least one of the generators iteratively until the required current is obtained at each of the electrodes.

2. Device according to claim 1, **characterised in that** the voltage generator controller comprises means for monitoring and/or varying the phase of the voltage supplied to each of the active electrodes.

3. Device according to either claim 1 or claim 2, **characterised in that** the voltage generator controller comprises means for monitoring and/or varying the frequency of the voltage supplied to each of the active electrodes.

4. Device according to any of the preceding claims, **characterised in that** through each of the active electrodes there is, at most, a current of 300 mA RMS.

5. Device according to any of the preceding claims, **characterised in that** for each of the voltage outputs of the voltage generators there is, at most, a voltage of 70 V RMS.

6. Device according to any of the preceding claims, **characterised in that** each of the outlets of the voltage generators has a maximum electric power of 50 W.

7. Device according to any of the preceding claims, **characterised in that** the signals generated by the voltage generators are sinusoidal signals, with a harmonic distortion of less than 50 %.

8. Device according to any of the preceding claims, **characterised in that** the signals generated by the voltage generators are signals with a frequency of between 100 kHz and 2 MHz.

9. Device according to any of the preceding claims, **characterised in that** the voltage generator controller comprises a microcontroller.

10. Device according to any of claims 1 to 8, **characterised in that** the voltage generator controller comprises a microprocessor.

11. Device according to any of claims 1 to 8, **characterised in that** the voltage generator controller comprises a programmable logic circuit.

12. Device according to any of claims 1 to 8, **characterised in that** the voltage generator controller is an analogue circuit.

13. Device according to any of the preceding claims, **characterised in that** at least one of the electrodes comprises a commutator which switches the active electrode from a first position connected to the output of the voltage generator to a second position connected to the return electrode.

14. Device according to any of the preceding claims, **characterised in that** at least one of the active electrodes comprises a temperature sensor.

15. Device according to any of the preceding claims, **characterised in that** the active electrodes comprise connection means to the patient.

**16.** Device according to claim 15, **characterised in that** said connection means to the patient are adhesive means.

**17.** Device according to claim 15, **characterised in that** said connection means to the patient are suction means.

**18.** Device according to any of claims 1 to 17, **characterised in that** said device comprises only one return electrode.

**Patentansprüche**

**1.** Elektrotherapiegerät, umfassend:

- mehrere aktive Elektroden;
- eine Gegenelektrode; und
- eine Mehrzahl von Spannungsgeneratoren, jeweils verbunden mit einer aktiven Elektrode;

wobei das Gerät eine Spannungsgenerator-Steuerung aufweist, die Mittel besitzt zum unabhängigen Überwachen und/oder Variieren der an jede der aktiven Elektroden angelegten Spannung; **dadurch gekennzeichnet, dass** die Gleichtaktspannung an dem gemeinsamen Widerstand dadurch reduziert wird, dass die Phase der Ströme der Elektroden modifiziert wird oder die Spannung von mindestens einem der Generatoren iterativ modifiziert wird, bis der erforderliche Strom an jeder der Elektroden erhalten wird.

**2.** Gerät nach Anspruch 1, **dadurch gekennzeichnet, dass** die Spannungsgenerator-Steuerung Mittel aufweist zum Überwachen und/oder Variieren der Phase der an jede der aktiven Elektroden angelegten Spannung.

**3.** Gerät nach Anspruch 1 oder 2, **dadurch gekennzeichnet, dass** die Spannungsgenerator-Steuerung Mittel aufweist zum Überwachen und/oder Variieren der Frequenz jede der aktiven Elektroden angelegten Spannung.

**4.** Gerät nach einem der vorhergehenden Ansprüche, **dadurch gekennzeichnet, dass** durch jede der aktiven Elektroden höchstens ein Strom von 300 mA (effektiv) fließt.

**5.** Gerät nach einem der vorhergehenden Ansprüche, **dadurch gekennzeichnet, dass** für jede der Ausgangsspannungen der Spannungsgeneratoren eine Höchstspannung von 70 V (effektiv) gilt.

**6.** Gerät nach einem der vorhergehenden Ansprüche, **dadurch gekennzeichnet, dass** jeder der Ausgänge der Spannungsgeneratoren eine elektrische Maximalleistung von 50 W aufweist.

**7.** Gerät nach einem der vorhergehenden Ansprüche, **dadurch gekennzeichnet, dass** die von den Spannungsgeneratoren erzeugten Signale Sinussignale mit einer Oberwellenverzerrung von weniger als 50% sind.

**8.** Gerät nach einem der vorhergehenden Ansprüche, **dadurch gekennzeichnet, dass** die von den Spannungsgeneratoren erzeugten Signale Signale mit einer Frequenz zwischen 100 kHz und 2 MHz sind.

**9.** Gerät nach einem der vorhergehenden Ansprüche, **dadurch gekennzeichnet, dass** die Spannungsgenerator-Steuerung einen Mikrocontroller aufweist.

**10.** Gerät nach einem der Ansprüche 1 bis 8, **dadurch gekennzeichnet, dass** die Spannungsgenerator-Steuerung einen Mikroprozessor aufweist.

**11.** Gerät nach einem der Ansprüche 1 bis 8, **dadurch gekennzeichnet, dass** die Spannungsgenerator-Steuerung eine programmierbare Logikschaltung aufweist.

**12.** Gerät nach einem der Ansprüche 1 bis 8, **dadurch gekennzeichnet, dass** die Spannungsgenerator-Steuerung eine Analogschaltung ist.

**13.** Gerät nach einem der vorhergehenden Ansprüche, **dadurch gekennzeichnet, dass** mindestens eine der Elektroden einen Kommutator aufweist, der die aktive Elektrode aus einer ersten Stellung verbunden mit dem Ausgang des Spannungsgenerators in eine zweite Stellung verbunden mit der Gegenelektrode umschaltet.

**14.** Gerät nach einem der vorhergehenden Ansprüche, **dadurch gekennzeichnet, dass** mindestens eine der aktiven Elektroden einen Temperatursensor besitzt.

**15.** Gerät nach einem der vorhergehenden Ansprüche, **dadurch gekennzeichnet, dass** die aktiven Elektroden Verbindungsmittel zu dem Patienten aufweisen.

**16.** Gerät nach Anspruch 15, **dadurch gekennzeichnet, dass** die Verbindungsmittel zu dem Patienten Klebemittel sind.

**17.** Gerät nach Anspruch 15, **dadurch gekennzeichnet, dass** die Verbindungsmittel zu dem Patienten Saugmittel sind.

**18.** Gerät nach einem der Ansprüche 1 bis 17, **dadurch gekennzeichnet, dass** das Gerät nur eine Gegenelektrode besitzt.

**Revendications**

**1.** Dispositif d'électrothérapie qui comprend :

- une pluralité d'électrodes actives ;
- une électrode de retour ; et
- une pluralité de générateurs de tension connectés chacun à une électrode active ;

ledit dispositif comprenant un contrôleur de générateur de tension qui a des moyens pour surveiller et/ou faire varier de façon indépendante la tension fournie à chacune des électrodes actives ;
**caractérisé en ce que** la tension en mode commun sur la résistance commune est réduite en modifiant la phase des courants des électrodes ou en modifiant la tension de l'un au moins des générateurs de façon itérative jusqu'à ce que le courant requis soit obtenu sur chacune des électrodes.

**2.** Dispositif selon la revendication 1, **caractérisé en ce que** le contrôleur de générateur de tension comprend des moyens pour surveiller et/ou faire varier la phase de la tension fournie à chacune des électrodes actives.

**3.** Dispositif selon l'une ou l'autre de la revendication 1 ou de la revendication 2, **caractérisé en ce que** le contrôleur de générateur de tension comprend des moyens pour surveiller et/ou faire varier la fréquence de la tension fournie à chacune des électrodes actives.

**4.** Dispositif selon l'une quelconque des revendications précédentes, **caractérisé en ce qu'**à travers chacune des électrodes actives circule, au maximum, un courant efficace de 300 mA.

**5.** Dispositif selon l'une quelconque des revendications précédentes, **caractérisé en ce que** pour chacune des sorties de tension des générateurs de tension, il y a au plus une tension efficace de 70 V.

**6.** Dispositif selon l'une quelconque des revendications précédentes, **caractérisé en ce que** chacune des sorties des générateurs de tension présente une puissance électrique maximale de 50 W.

**7.** Dispositif selon l'une quelconque des revendications précédentes, **caractérisé en ce que** les signaux générés par les générateurs de tension sont des signaux sinusoïdaux, avec une distorsion harmonique inférieure à 50 %.

**8.** Dispositif selon l'une quelconque des revendications précédentes, **caractérisé en ce que** les signaux générés par les générateurs de tension sont des signaux dont la fréquence est comprise entre 100 kHz et 2 MHz.

**9.** Dispositif selon l'une quelconque des revendications précédentes, **caractérisé en ce que** le contrôleur de générateur de tension comprend un microcontrôleur.

**10.** Dispositif selon l'une quelconque des revendications 1 à 8, **caractérisé en ce que** le contrôleur de générateur de tension comprend un microprocesseur.

**11.** Dispositif selon l'une quelconque des revendications 1 à 8, **caractérisé en ce que** le contrôleur de générateur de tension comprend un circuit logique programmable.

**12.** Dispositif selon l'une quelconque des revendications 1 à 8, **caractérisé en ce que** le contrôleur de générateur de tension est un circuit analogique.

**13.** Dispositif selon l'une quelconque des revendications précédentes, **caractérisé en ce que** l'une au moins des électrodes comprend un commutateur qui commute l'électrode active depuis une première position connectée à la sortie du générateur de tension jusqu'à une deuxième position connectée à l'électrode de retour.

**14.** Dispositif selon l'une quelconque des revendications précédentes, **caractérisé en ce que** l'une au moins des électrodes actives comprend un capteur de température.

**15.** Dispositif selon l'une quelconque des revendications précédentes, **caractérisé en ce que** les électrodes actives comprennent des moyens de connexion au patient.

**16.** Dispositif selon la revendication 15, **caractérisé en ce que** lesdits moyens de connexion au patient sont des moyens adhésifs.

**17.** Dispositif selon la revendication 15, **caractérisé en ce que** lesdits moyens de connexion au patient sont des moyens d'aspiration.

**18.** Dispositif selon l'une quelconque des revendications 1 à 17, **caractérisé en ce que** ledit dispositif comprend seulement une électrode de retour.

*Fig.1*

*Fig.2*

*Fig.3*

Fig.4

*Fig. 5*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 287964
- EP 0893140 A
- US 20080015572 A
- ES 1030072
- ES 2304272
- US 20020082653 A
- US 2003139781 A1
- US 2010152817 A1
- WO 2013059158 A1